# EUROPEAN PATENT SPECIFICATION

(11) **EP 3 478 277 B1**
(45) Date of publication and mention of the grant of the patent: **23.06.2021**
(21) Application number: 17807363.1
(22) Date of filing: 31.05.2017
(51) Int. Cl.: A61K 31/095, A61K 33/04, A61K 31/455, A61K 31/355, A61K 31/203, A61K 31/07, A61K 8/23, A61K 8/34, A61P 17/10, A61Q 19/08, A61K 8/67, A61Q 19/00

(54) **ANTI-ACNE COMPOSITIONS AND METHODS OF USE**
ANTI-AKNE-ZUSAMMENSETZUNGEN UND VERFAHREN ZUR VERWENDUNG
COMPOSITIONS ANTI-ACNÉ ET PROCÉDÉS D'UTILISATION

(30) Priority: 31.05.2016 US 201662343479 P
(43) Date of publication of application: 08.05.2019
(73) Proprietor: L'OREAL, 75008 Paris (FR)
(72) Inventor: ROBERT, Valerie, A., Clark, NJ 07066 (US)
(74) Representative: Lavoix
(86) International application number: PCT/US2017/035095
(87) International publication number: WO 2017/210239

(56) References cited:
- FR-A1- 3 018 189
- US-A1- 2010 129 465
- US-A1- 2013 289 102
- US-A1- 2015 024 077
- US-A1- 2015 064 122
- US-A1- 2015 335 560
- US-A1- 2015 342 990

## Description

### FIELD OF THE DISCLOSURE

The present disclosure relates cosmetic compositions and methods for using the cosmetic compositions to improve the appearance of skin. For example, the cosmetic compositions can be used to treat acne or to provide anti-aging benefits to the skin.

### BACKGROUND

Acne often affects teenagers but it is also problematic in adults. Although the precise causes for the development of acne have not been fully resolved, it is believed that a combination of the following factors is involved: (a) excessive production of sebum; (b) blocking of the entrance of the hair follicle canals; and (c) inflammatory processes caused by excessive development of bacteria. One of the causes for excessive sebum production is the increased activity of androgenic hormones, which stimulate the sebaceous glands in order to raise the production of sebum. The inflammatory response is a reaction to the follicle canal blocking by keratin plugs. Hyperkeratinization of the follicular duct is observed simultaneously, creating in the pilosebaceous follicle an environment which is rich in nutrients for the bacterial flora and which especially promotes the proliferation of diphtheroid anaerobic germs such as the Propionibacteria (acnes, granulosnm, avidum) and, consequently, inflammation of the skin.

The basic lesion of acne is the microcomedo. Accumulation of sebum and keratinous debris results in a visible closed comedo, or whitehead, and its continued distension causes an open comedo, or blackhead. The dark color of blackheads is due to oxidized melanin. Blackheads and microcysts are noninflammatory lesions of acne, but some comedones evolve into inflammatory papules, pustules, or nodules, and can become chronic granulomatous lesions. The initial inflammatory cell in an acute acne papule is the CD4+T lymphocyte. Duct rupture is not a prerequisite for inflammation, which is due to the release of pro-inflammatory substances from the duct. When inflammation develops, neutrophil chemotaxis occurs. These neutrophils secrete hydrolytic enzymes that cause further damage and increased permeability of the follicular wall. In pustules, neutrophils are present much earlier. More persistent lesions exhibit granulomatous histology that can lead to scarring.

The aims of treating acne are to minimize the number and severity of lesions, prevent scarring, limit disease duration, and reduce the social and psychological stress that affects many patients, particularly teenagers. Conventional treatment is directed at correcting the three major factors that seem to cause acne: (1) androgenic stimulation of the sebaceous glands and increased sebum production; (2) abnormal keratinization and impaction in the pilosebaceous canal causing obstruction to sebum flow; and (3) proliferation of *P. acnes.* Thus, topical agents that remove comedones, such as topical retinoids are particularly effective because they normalize desquamination within the follicular orifice, which allows the sebum to flow freely onto the surface of the skin; adalpalene, tretinoin, and tazarotene have been shown to have efficacy in treating mild to moderate acne, but all three have reported to have skin-irritating side effects including erythema, pruritis, burning/stinging, and scaling/flaking. The side effects of retinoid use are so extreme that many individuals cannot tolerate topical application of these agents at all.

Salicylic acid and benzoyl peroxide have been used to treat acne for some time. Both agents dry the skin, which helps in acne management, but they may cause some skin irritation in perilesional skin areas of acne patients, especially patients with sensitive skin, and in some cases the erythema is extreme. Moreover, it has been reported that benzoyl peroxide seems to induce free radical production that can produce skin changes that qualitatively resemble ultraviolet B damage, e.g., increases in epidermal thickness, and deleterious changes in elastin and glycosaminoglycan content. See Ibbotson, et al., The Effects of Radicals Compared with UVB as Initiating Species for the Induction of Chronic Cutaneous Photodamage, J. INVEST. DERMATOL., 1999, June;112(6): 933 938. Topical and oral antibiotics (especially tetracycline, erythromycin, and clindamycin) are sometimes prescribed for patients with inflammatory papules and pustules, but, in addition to the undesirability of antibiotic overuse in general, which can lead to enhanced susceptibility to infection, disadvantages to such treatments include phototoxicity and interactions with other medications.

Other factors that play a role in exacerbating acne, including oil-based cosmetics and some drugs (e.g., androgenic hormones, high-progestin birth control pills, systemic corticosteroids, and iodide- and bromide-containing agents) are often minimized during acne treatment.

Document US2010/0129465 discloses methods and compositions for treating acne. The compositions may comprise sulfur and niacinamide.

### SUMMARY OF THE DISCLOSURE

The present disclosure relates to cosmetic compositions and methods for using the cosmetic compositions to improve the appearance of skin as defined in the appended set of claims. For example, the cosmetic compositions can be used to treat acne, to treat inflammation, to provide anti-aging benefits to the skin, etc. The cosmetic compositions comprise: (a) a cosmetically acceptable carrier comprising water and one or more cosmetically acceptable organic solvents; and (b) a combination of skin-active ingredients comprising:
i. 5 wt % to 15 wt % of sulfur;
ii. 1 wt % to 10 wt % of niacinamide
iii. 0.1wt % to 2 wt % of phenylethyl resorcinol;
iv. glycolic acid;
v. allantoin; and
(c) one or more anionic surfactants, wherein the total amount of the one or more anionic surfactants of (c) is from 0.1 wt % to 15 wt %, based on the total weight of the cosmetic composition.

The cosmetic compositions include one or more nonionic surfactants. Non-limiting examples of nonionic surfactants include polyol esters, glycerol ethers, oxyethylenated ethers, oxypropylenated ethers, and ethylene glycol polymers. Combinations of nonionic surfactants may be employed, for example, a combination of glyceryl stearate, PEG-100 stearate, and ceteareth-20.

The one or more nonionic surfactants may comprise one or more fatty alcohols. For example, the one or more fatty alcohols may be selected from the group consisting of selected from the group consisting of caproic alcohol, caprylic alcohol, 2-ethylhexyl alcohol, capric alcohol, lauryl alcohol, isotridecyl alcohol, myristyl alcohol, cetyl alcohol, hexyl decanol, palmitoleyl alcohol, stearyl alcohol, cetearyl alcohol, isostearyl alcohol, oleyl alcohol, elaidyl alcohol, petroselinyl alcohol, linolyl alcohol, linolenyl alcohol, elaeostearyl alcohol, arachyl alcohol, gadoleyl alcohol, behenyl alcohol, octyl dodecanol, erucyl alcohol brassidyl alcohol, coconut oil, behenyl alcohol, and mixtures thereof.

Vitamins, especially vitamins that are helpful to the health and vitality of skin may be included in the cosmetic compositions. For example, the vitamin(s) may include ascorbic acid, vitamin A, vitamin E, and/or vitamin B₅.

The instant disclosure further relates to methods for treating acne, methods for providing anti-inflammatory and/or antibacterial benefits to the skin, and to methods for improving the appearance of skin. The methods include application of the cosmetic compositions described herein to the skin. Often the methods relate to treating an individual in need of treatment, e.g., an individual suffering from acne, and individual prone to acne breakouts, an individual suffering from acne scarring, and/or and individual suffering from post-inflammatory hyperpigmentation.

### DETAILED DESCRIPTION OF THE DISCLOSURE

The present disclosure relates to cosmetic compositions and methods for using the cosmetic compositions to improve the appearance of skin. The compositions comprise:
(a) a cosmetically acceptable carrier comprising water and one or more cosmetically acceptable organic solvents; and
(b) a combination of skin-active ingredients comprising:
   i. 5 wt.% to 15 wt.% of sulfur;
   ii. 1 wt.% to 10 wt.% of niacinamide;
   iii. 0.1 wt.% to 2 wt.% of phenylethyl resorcinol;
   iv. glycolic acid;
   v. allantoin; and
(c) one or more nonionic surfactants, wherein the total amount of the one or more nonionic surfactants of (c) is from 0.1 wt.% to 15 wt.%, based on the total weight of the cosmetic composition.

The total amount of sulfur is from 5 wt.% to 15 wt.% based on the total weight of the cosmetic composition. In some cases the total amount of sulfur in the compositions is higher than the total amount of all other skin-active ingredients combined. Although the compositions include sulfur, the compositions are unique in that they do not exhibit an objectionable odor that is common amongst sulfur containing products. Without wishing to be bound by any particular theory, applicants believe that use of a starch, such as corn starch, contributes to the lack of an objectionable odor.

The total amount of niacinamide is from 1 wt.% to 10 wt.%, based on the total weight of the cosmetic composition. The total amount of the niacinimade may be 1 wt.% to 5 wt.%, 6 wt.%, 7 wt.%, 8 wt.% 9 wt.%. Likewise, the total amount of niacinamide may be 2 wt.%, or 3 wt.%, to 8 wt.%.

The total amount of phenylethyl resorcinol is from 0,1 wt.% to 2 wt.%, based on the total weight of the cosmetic composition. Likewise, the total amount of the phenylethyl resorcinol may be 0.1 wt.% to 1 wt. %.

The total amount of glycolic acid, when present may be 1 wt.% to 10 wt.%, based on the total weight of the cosmetic composition. The total amount of the glycolic acid may be 1 wt.% to 5 wt.%, 6 wt.%, 7 wt.%, 8 wt.% 9 wt.%. Likewise, the total amount of glycolic acid may be 2 wt.%, or 3 wt.%, to 8 wt.%.

The total amount of allantoin, when present, may be 0.01 wt.% to 5 wt.%, based on the total weight of the cosmetic composition. The total amount of the allantoin may be 0.01 wt.% to 1 wt.%, 2 wt.%, 3 wt.%, or 4 wt.%. Likewise, the total amount of the allantoin may be 0.05 wt.% to 1 wt.%, 2 wt.%, 3 wt.%, 4 wt.%, or 5 wt.%.

As mentioned above, the skin-active ingredients are provided in a cosmetically acceptable carrier. The cosmetically acceptable carrier includes water; and additionally include one or more cosmetically acceptable organic solvents. Examples of cosmetically acceptable organic solvents include ethylene glycol, propylene glycol, butylene glycol, hexylene glycol, propane diol, and glycerin.

The cosmetic compositions include one or more nonionic surfactants. Examples of nonionic surfactants include polyol esters, glycerol ethers, oxyethylenated ethers, oxypropylenated ethers, and ethylene glycol polymers. Combinations of nonionic surfactants may be employed, for example, a combination of a polyol ester and an ethylene glycol polymer; more specifically, a combination of glyceryl stearate, PEG-100 stearate, and ceteareth-20.

The one or more nonionic surfactant may include one or more fatty alcohols. For example, the one or more fatty alcohols may be selected from the group consisting of selected from the group consisting of caproic alcohol, caprylic alcohol, 2-ethylhexyl alcohol, capric alcohol, lauryl alcohol, isotridecyl alcohol, myristyl alcohol, cetyl alcohol, hexyl decanol, palmitoleyl alcohol, stearyl alcohol, cetearyl alcohol, isostearyl alcohol, oleyl alcohol, elaidyl alcohol, petroselinyl alcohol, linolyl alcohol, linolenyl alcohol, elaeostearyl alcohol, arachyl alcohol, gadoleyl alcohol, behenyl alcohol, octyl dodecanol, erucyl alcohol brassidyl alcohol, coconut oil, behenyl alcohol, and mixtures thereof.

The total amount of the one or more nonionic surfactants is 0.1 wt.% to 15 wt.%, based on the total weight of the cosmetic composition. The total amount of the one or more nonionic surfactants may be 1 wt.% to 6 wt.%, 8 wt.%, 10 wt.%, 12 wt.%, or 14 wt.%. Likewise, the total amount of the one or more nonionic surfactants may be 2 wt.% to 6 wt.%, 8 wt.%, 10 wt.%, 12 wt.%, or 14 wt.%.

One or more vitamins, especially vitamins that are helpful to the health and vitality of skin, may be included in the cosmetic compositions. For example, the vitamin(s) may include ascorbic acid, vitamin A, vitamin E, and/or vitamin B₅. The total amount of the one or more vitamins are typically about 0.01 wt.% to about 5 wt.%, based on the total weight of the cosmetic composition. In some cases, the total amount of the one or more vitamins are 0.1 wt.% to 1 wt.%, 2 wt.%, 3 wt.%, 4 wt.%, or 5 wt.%.

The instant disclosure further relates to methods for treating acnes, methods for providing anti-inflammatory and/or antibacterial benefits to the skin, and to methods for improving the appearance of skin. The methods include application of the cosmetic compositions described herein to the skin. Often the methods relate to treating an individual in need of treatment, e.g., an individual suffering from acne or prone to acne breakouts. The compositions may be applied daily to the skin, especially to the face, neck, back, and or shoulders. The compositions may be applied once a day or more often, for example two or three times per day. In some cases, the compositions are applied in the morning. In some cases the composition are applied in the evening. Furthermore, in some instances, the compositions may be applied both in the morning and again in the evening. The compositions may be applied daily for multiple days, weeks, or even months. In some cases, the treatment may be brief in order to address a particular break-out, for example, for one day to one week (7 days). The treatment may be at least one week and extend for two, three, or four weeks, or longer.

More exhaustive lists of components useful in the compositions disclosed herein are presented below.

### Cosmetically Acceptable Carrier (or Solvent)

The compositions of the present disclosure are presented in a cosmetically acceptable solvent. This cosmetically acceptable solvent comprises a mixture of water and at least one cosmetically acceptable organic solvent.

As examples of organic solvents, mentions can be made of monoalcohols and polyols such as ethyl alcohol, isopropyl alcohol, propyl alcohol, benzyl alcohol, and phenylethyl alcohol, or glycols or glycol ethers such as, for example, monomethyl, monoethyl and monobutyl ethers of ethylene glycol, propylene glycol or ethers thereof such as, for example, monomethyl ether of propylene glycol, butylene glycol, hexylene glycol, dipropylene glycol as well as alkyl ethers of diethylene glycol, for example monoethyl ether or monobutyl ether of diethylene glycol.

Other suitable examples of organic solvents are ethylene glycol, propylene glycol, butylene glycol, hexylene glycol, propane diol, and glycerin.

The organic solvents for use in the present disclosure can be volatile or nonvolatile compounds.

The cosmetically acceptable solvent may be employed according to the present disclosure in an amount ranging from about 5% to about 95% by weight, or such as from about 20% to about 90% by weight, such as from about 30 to about 80% by weight, or such as from about 35% to about 75% by weight, such as from about 5 to about 50% by weight, such as from about 50 to 95% by weight, based on the total weight of the composition.

The organic solvent may be employed according to the present disclosure in an amount ranging from about 0.1% to about 25% by weight, such as from about 1% to about 15% by weight, or such as from about 3% to about 10% by weight, or such as from about 5% to about 10% by weight, based on the total weight of the composition of the present disclosure.

### Skin-Active Ingredients

In addition to the skin-active ingredients already described, the compositions of the instant disclosure may contain addition skin-active ingredient such as a humectant and moisturizing ingredients, an anti-aging agent, a depigmenting agent, an anti-wrinkle agent, or an agent that treats oily skin. Non-limiting examples of the one or more active agents include adenosine, 2-[4-(2-hydroxyethyl)piperazin-1-yl]ethanesulfonic acid (HEPES), hyaluronic acid, lanolin, citric acid, malic acid, lactic acid, tartaric acid, salicylic acid, vitamin C, a vitamin, a retinoid, retinal, retinoic acid, a carotenoid, an amino acid, a protein, an enzyme, and a coenzyme. In some cases the active ingredient is adenosine.

Humectants and moisturizing ingredients may be in particular glycerol and its derivatives, urea and its derivatives, especially Hydrovance marketed by National Starch, lactic acid, hyaluronic acid, AHA, BHA, sodium pidolate, xylitol, serine, sodium lactate, ectoin and its derivatives, chitosan and its derivatives, collagen, plankton, an extract of Imperata cylindra sold under the name Moist 24 by Sederma, homopolymers of acrylic acid as Lipidure-HM of NOF Corporation, beta-glucan and in particular sodium carboxymethyl beta-glucan Mibelle-AG-Biochemistry, a mixture of oils passionflower, apricot, corn, and rice bran sold by Nestle under the name NutraLipids, a C-glycoside derivatives, in particular the C-13-D-xylopyranoside-2-hydroxypropane in the form of a solution at 30% by weight of active material in a water/propylene glycol mixture (60/40 wt %) as the product produced by the company Chimex under the trade name "Mexoryl SBB", a rose hip oil marketed by Nestle, a micro-algae extract Prophyridium cruentum enriched with zinc, marketed under the name by Vincience Algualane Zinc spheres of collagen and chondroitin sulfate of marine origin (Atelocollagen) sold by the company Engelhard Lyon under the name Marine Filling Spheres, hyaluronic acid spheres such as those marketed by Engelhard Lyon, and arginine.

Depigmenting agents include vitamin C and its derivatives and especially vitamin CG, CP and 3-O ethyl vitamin C, alpha and beta arbutin, ferulic acid, lucinol and its derivatives, kojic acid, resorcinol and derivatives thereof, tranexamic acid and derivatives thereof, gentisic acid, homogentisic, methyl gentisate or homogentisate, dioic acid, D pantheteine calcium sulphonate, lipoic acid, ellagic acid, vitamin B3, linoleic acid and its derivatives, ceramides and their counterparts, derived from plants such as chamomile, bearberry, the aloe family (vera, ferox, bardensis), mulberry, skullcap, a water kiwi fruit (Actinidia chinensis) marketed by Gattefosse, an extract of Paeonia suffruticosa root, such as that sold by Ichimaru Pharcos under the name Liquid Botanpi Be an extract of brown sugar (Saccharum officinarum) such as molasses extract marketed by Taiyo Kagaku under the name Liquid Molasses, without this list being exhaustive. Particular depigmenting agents include vitamin C and its derivatives and especially vitamin CG, CP and 3-O ethyl vitamin C, alpha and beta arbutin, ferulic acid, kojic acid, resorcinol and derivatives, D pantheteine calcium sulfonate, lipoic acid, ellagic acid, vitamin B3, a water kiwi fruit (Actinidia chinensis) marketed by Gattefosse, an extract of Paeonia suffruticosa root, such as that sold by the company Ichimaru Pharcos under the name Botanpi Liquid B.

The term "anti-wrinkle active" refers to a natural or synthetic compound producing a biological effect, such as the increased synthesis and/or activity of certain enzymes, when brought into contact with an area of wrinkled skin, this has the effect of reducing the appearance of wrinkles and/or fine lines. Exemplary anti-wrinkle actives may be chosen from: desquamating agents, anti-glycation agents, inhibitors of NO-synthase, agents stimulating the synthesis of dermal or epidermal macromolecules and/or preventing their degradation, agents for stimulating the proliferation of fibroblasts and/or keratinocytes, or for stimulating keratinocyte differentiation reducing agents; muscle relaxants and/or dermo-decontracting agents, anti-free radical agents, and mixtures thereof.

Examples of such compounds are: adenosine and its derivatives and retinol and its derivatives such as retinol palmitate, ascorbic acid and its derivatives such as magnesium ascorbyl phosphate and ascorbyl glucoside; tocopherol and derivatives thereof such as tocopheryl acetate, nicotinic acid and its precursors such as nicotinamide; ubiquinone; glutathione and precursors thereof such as L-2-oxothiazolidine-4-carboxylic acid, the compounds C-glycosides and their derivatives as described in particular in EP-1345919, in particular C-beta-D-xylopyranoside-2-hydroxy-propane as described in particular in EP-1345919, plant extracts including sea fennel and extracts of olive leaves, as well as plant and hydrolysates thereof such as rice protein hydrolysates or soybean proteins; algal extracts and in particular laminaria, bacterial extracts, the sapogenins such as diosgenin and extracts of Dioscorea plants, in particular wild yam, comprising: the a-hydroxy acids, f3-hydroxy acids, such as salicylic acid and n-octanoyl-5-salicylic oligopeptides and pseudodipeptides and acyl derivatives thereof, in particular acid {2-[acetyl-(3-trifluoromethyl-phenyl)-amino]-3-methyl-}acetic acid and lipopeptides marketed by the company under the trade names SEDERMA Matrixyl 500 and Matrixyl 3000; lycopene, manganese salts and magnesium salts, especially gluconates, and mixtures thereof.

As adenosine derivatives include especially non-phosphate derivatives of adenosine, such as in particular the 2'-deoxyadenosine, 2',3'-adenosine isopropoylidene; the toyocamycine, 1-methyladenosine, N-6-methyladenosine; adenosine N-oxide, 6-methylmercaptopurine riboside, and the 6-chloropurine riboside.

Other derivatives include adenosine receptor agonists such as adenosine adenosine phenylisopropyl ("PIA"), 1-methylisoguanosine, N6-cyclohexyladenosine (CHA), N6-cyclopentyladenosine (CPA), 2-chloro-N6-cyclopentyladenosine, 2-chloroadenosine, N6-phenyladenosine, 2-phenylaminoadenosine, MECA, N 6-phenethyladenosine, 2-p-(2-carboxy-ethyl) phenethyl-amino-5'- -N-ethylcarboxamido adenosine (CGS-21680), N-ethylcarboxamido-adenosine (NECA), the 5'(N-cyclopropyl)-carboxamidoadenosine, DPMA (PD 129.944) and metrifudil.

In some instances, the compositions of the instant disclosure comprise a skin-active ingredient that addresses oily skin. These actives can be sebo-regulating or antiseborrhoeic agents capable of regulating the activity of sebaceous glands. These include: retinoic acid, benzoyl peroxide, sulfur, vitamin B6 (pyridoxine or) chloride, selenium, samphire--the cinnamon extract blends, tea and octanoylglycine such as--15 Sepicontrol A5 TEA from Seppic--the mixture of cinnamon, sarcosine and octanoylglycine marketed especially by Seppic under the trade name Sepicontrol A5--zinc salts such as zinc gluconate, zinc pyrrolidonecarboxylate (or zinc pidolate), zinc lactate, zinc aspartate, zinc carboxylate, zinc salicylate 20, zinc cysteate;--derivatives particularly copper and copper pidolate as Cuivridone Solabia- -extracts from plants of Arnica montana, Cinchona succirubra, Eugenia caryophyllata, Humulus lupulus, Hypericum perforatum, Mentha pipenta 25 Rosmarinus officinalis, Salvia officinalis and Thymus vulgaris, all marketed for example by Maruzen-extracts of meadowsweet (Spiraea ulmaria), such as that sold under the name Sebonormine by Silab--extracts of the alga Laminaria saccharina, such as that sold under the 30 name Phlorogine by Biotechmarine--the root extracts of burnet mixtures (Sanguisorba officinalis/Poterium officinale), rhizomes of ginger (Zingiber officinalis) and cinnamon bark (Cinnamomum cassia), such as that sold under the name Sebustop by Solabia--extracts of flaxseed such as that sold under the name Linumine by Lucas Meyer--Phellodendron extracts such as those sold under the name Phellodendron extract BG by Maruzen or Oubaku liquid B by Ichimaru Pharcos--of argan oil mixtures extract of Serenoa serrulata (saw palmetto) extract and sesame seeds such as that sold under the name Regu SEB by Pentapharmmixtures of extracts of willowherb, of Terminalia chebula, nasturtium and of bioavailable zinc (microalgae), such as that sold under the name Seborilys Green Tech;--extracts of Pygeum afrianum such as that sold under the name Pygeum afrianum sterolic lipid extract by Euromed--extracts of Serenoa serrulata such as those sold under the name Viapure Sabal by Actives International, and those sold by the company Euromed--of extracts of plantain blends, Berberis aquifolium and sodium salicylate 20 such as that sold under the name Seboclear Rahn--extract of clove as that sold under the name Clove extract powder by Maruzen--argan oil such as that sold under the name Lipofructyl Laboratories Serobiologiques; 25--lactic protein filtrates, such as that sold under the name Normaseb by Sederma--the seaweed laminaria extracts, such as that sold under the name Laminarghane by Biotechmarine--oligosaccharides seaweed Laminaria digitata, such as that sold under the name Phycosaccharide 30 AC by the company Codif--extracts of sugar cane such as that sold under the name Policosanol by the company Sabinsa, the sulfonated shale oil, such as that sold under the name Ichtyol Pale by Ichthyol-extracts of 'meadowsweet (Spiraea ulmaria) such as that sold under the name Cytobiol Ulmaire by societeLibiol--sebacic acid, especially sold in the form of a sodium polyacrylate gel under the name Sebosoft by Sederma--glucomannans extracted from konjac tuber and modified with alkylsulfonate chains such as that sold under the name Biopol Beta by Arch Chemical--extracts of Sophora angustifolia, such as those sold under the name Sophora powder or Sophora extract by Bioland-extracts of cinchona bark succirubra such as that sold under the name Red Bark HS by Alban Muller--extracts of Quillaja saponaria such as that sold under the name 15 Panama wood HS by Alban Muller--glycine grafted onto an undecylenic chain, such as that sold under the name Lipacide UG OR by SEPPIC--the mixture of oleanolic acid and nordihydroguaiaretic acid, such as that sold under the form of a gel under the name AC.Net by Sederma; 20--phthalimidoperoxyhexanoic acid--citrate tri (C12-C13) sold under the name COSMACOL.RTM. ECI by Sasol; trialkyl citrate (C14-C15) sold under the name COSMACOL.RTM. ECL by Sasol--10-hydroxydecanoic acid, including mixtures acid-hydroxydecanoic October 25, sebacic acid and 1,10-decandiol such as that sold under the name Acnacidol BG by Vincience and mixtures thereof.

### Preservatives

One or more preservatives may be included in the compositions described herein for treating hair. Suitable preservatives include, but are not limited to, glycerin containing compounds (e.g., glycerin and/or ethylhexylglycerin and/or phenoxyethanol), benzyl alcohol, parabens (methylparaben, ethylparaben, propylparaben, butylparaben, isobutylparaben, etc.), sodium benzoate, ethylenediamine-tetraacetic acid (EDTA), potassium sorbate, and/or grapefruit seed extract, or combinations thereof. More than one preservative may be included in the composition. Other preservatives are known in the cosmetics industries and include salicylic acid, DMDM Hydantoin, Formaldahyde, Chlorphenism, Triclosan, Imidazolidinyl Urea, Diazolidinyl Urea, Sorbic Acid, Methylisothiazolinone, Sodium Dehydroacetate, Dehydroacetic Acid, Quaternium-15, Stearalkonium Chloride, Zinc Pyrithione, Sodium Metabisulfite, 2-Bromo-2-Nitropropane, Chlorhexidine Digluconate, Polyaminopropyl biguanide, Benzalkonium Chloride, Sodium Sulfite, Sodium Salicylate, Citric Acid, Neem Oil, Essential Oils (various), Lactic Acid, and Vitamin E (tocopherol).

The preservative is optionally included in an amount ranging from about 0.01 wt.% to about 5 wt.%, about 0.05% to about 4 wt.%, or about 0.06 wt.% to about 3 wt.%, based on the total weight of the composition.

### Thickeners

The cosmetic compositions described herein may include one or more thickeners. The thickeners may be in an amount of 0.1 wt.% to 20 wt.%, 0.1 to 10 wt.%, 0.1 wt.% to 9 wt.%, 0.2 wt.% to 9 wt.%, 0.3 wt.% to 9 wt.%, 0.4 wt.% to 8 wt.%, 0.5 wt.% to 5 wt.%, 1 wt.% to 5 wt.%, or 2 wt.% to 4 wt.%. Further, the amount of thickener may be from 0.1, 0.2, 0.3, 0.4, 0.5, 0.6, 0.7, 0.8, 0.9, 1.0, or 1.5 wt.% to 2, 3, 4, 5, 6, 7, 8, 9, 10, 15, or 20 wt.%.

The one or more thickeners may be xanthan gum, guar gum, biosaccharide gum, cellulose, acacia Seneca gum, sclerotium gum, agarose, pechtin, gellan gum, hyaluronic acid. Additionally, the one or more thickeners may include polymeric thickeners selected from the group consisting of ammonium polyacryloyldimethyl taurate, ammonium acryloyldimethyltaurate/VP copolymer, sodium polyacrylate, acrylates copolymers, polyacrylamide, carbomer, and acrylates/C10-30 alkyl acrylate crosspolymer. In some cases, the composition includes ammonium polyacryloyldimethyl taurate and/or sodium polyacrylate.

Many thickeners are water-soluble, and increase the viscosity of water or form an aqueous gel when the cosmetic composition of the invention is dispersed/dissolved in water. The aqueous solution may be heated and cooled, or neutralized, for forming the gel, if necessary. The thickener may be dispersed/dissolved in an aqueous solvent that is soluble in water, e.g., ethyl alcohol when it is dispersed/dissolved in water. Non-limiting examples of various types of thickeners include:

### a. Carboxylic Acid Polymers

These polymers are crosslinked compounds containing one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol.

Examples of commercially available carboxylic acid polymers useful herein include the carbomers, which are homopolymers of acrylic acid crosslinked with allyl ethers of sucrose or pentaerytritol. The carbomers are available as the Carbopol.RTM. 900 series from B.F. Goodrich (e.g., Carbopol® 954). In addition, other suitable carboxylic acid polymeric agents include Ultrez® 10 (B.F. Goodrich) and copolymers of C10-30 alkyl acrylates with one or more monomers of acrylic acid, methacrylic acid, or one of their short chain (i.e., C1-4 alcohol) esters, wherein the crosslinking agent is an allyl ether of sucrose or pentaerytritol. These copolymers are known as acrylates/C10-C30 alkyl acrylate crosspolymers and are commercially available as Carbopol.RTM. 1342, Carbopol® 1382, Pemulen TR-1, and Pemulen TR-2, from B.F. Goodrich. In other words, examples of carboxylic acid polymer thickeners useful herein are those selected from carbomers, acrylates/C10-C30 alkyl acrylate crosspolymers, and mixtures thereof.

### b. Crosslinked Polyacrylate Polymers

The compositions of the present disclosure can optionally contain crosslinked polyacrylate polymers useful as thickeners or gelling agents including both cationic and nonionic polymers. Examples of useful crosslinked nonionic polyacrylate polymers and crosslinked cationic polyacrylate polymers are those described in U.S. Pat. No. 5,100,660, U.S. Pat. No. 4,849,484, U.S. Pat. No. 4,835,206, U.S. Pat. No. 4,628,078 U.S. Pat. No. 4,599,379 and EP 228,868.

### c. Polyacrylamide Polymers

The compositions of the present disclosure can optionally contain polyacrylamide polymers, especially nonionic polyacrylamide polymers including substituted branched or unbranched polymers. Among these polyacrylamide polymers is the nonionic polymer given the CTFA designation polyacrylamide and isoparaffin and laureth-7, available under the Tradename Sepigel 305 from Seppic Corporation.

Other polyacrylamide polymers useful herein include multi-block copolymers of acrylamides and substituted acrylamides with acrylic acids and substituted acrylic acids. Commercially available examples of these multi-block copolymers include Hypan SR150H, SS500V, SS500W, SSSA100H, from Lipo Chemicals, Inc.

The compositions may also contain thickening and texturising gels of the type as exemplified by the product range called Lubrajel® from United Guardian. These gels have moisturizing, viscosifying, stabilizing properties.

### d. Polysaccharides

A wide variety of polysaccharides can be useful herein. "Polysaccharides" refer to gelling agents that contain a backbone of repeating sugar (i.e., carbohydrate) units. Nonlimiting examples of polysaccharide gelling agents include those selected from the group consisting of cellulose, carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof. Also useful herein are the alkyl-substituted celluloses. Preferred among the alkyl hydroxyalkyl cellulose ethers is the material given the CTFA designation cetyl hydroxyethylcellulose, which is the ether of cetyl alcohol and hydroxyethylcellulose. This material is sold under the tradename Natrosol® CS Plus from Aqualon Corporation.

Other useful polysaccharides include scleroglucans comprising a linear chain of (1-3) linked glucose units with a (1-6) linked glucose every three units, a commercially available example of which is Clearogel™. CS11 from Michel Mercier Products Inc.

### e. Gums

Other thickening and gelling agents useful herein include materials which are primarily derived from natural sources. Nonlimiting examples of these gelling agent gums include acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluronic acid, hydrated silica, hydroxypropyl chitosan, hydroxypropyl guar, karaya gum, kelp, locust bean gum, natto gum, potassium alginate, potassium carrageenan, propylene glycol alginate, sclerotium gum, sodium carboxymethyl dextran, sodium carrageenan, tragacanth gum, xanthan gum, and mixtures thereof.

Additional examples of water-soluble thickeners include water-soluble natural polymers, water-soluble synthetic polymers, clay minerals and silicic anhydride. Non-limiting examples of water-soluble natural polymers include gum arabic, tragacanth gum, karaya gum, guar gum, gellan gum, tara gum, locust bean gum, tamarind gum, sodium alginate, alginic acid propyleneglycol ester, carrageenan, farcelluran, agar, high-methoxy pectin, low-methoxy pectin, xanthine, chitosan, starch (for example starch derived from corn, potato, wheat, rice, sweet potato and tapioca, a-starch, soluble starch), fermentation polysaccharide (for example, xanthan gum, pullulan, carciran, dextran), acidic hetero-polysaccharide derived form callus of plants belonging to Polyantes sp. (for example, tuberous polysaccharide), proteins (for example, sodium casein, gelatin, albumin), chondroitin sulfate, and hyaluronic acid.

Non-limiting examples of water-soluble synthetic polymers include polyvinyl alcohol, sodium polyacrylate, sodium polymethacrylate, polyacrylic acid glycerin ester, carboxyvinyl polymer, polyacrylamide, polyvinyl pyrrolidone, polyvinyl methylether, polyvinyl sulfone, maleic acid copolymer, polyethylene oxide, polydiallyl amine, polyethylene imine, water soluble cellulose derivatives (for example, carboxymethyl cellulose, methyl cellulose, methylhydroxypropyl cellulose, hydroxyethyl cellulose, hydroxypropyl cellulose, cellulose sulfate sodium salt), and starch derivatives (for example, starch oxide, dialdehyde starch, dextrin, British gum, acetyl starch, starch phosphate, carboxymethyl starch, hydroxyethyl starch, hydroxypropyl starch).

### Surfactants

The various compositions described herein may include one or more surfactants, including cationic, anionic, non-ionic and/or amphoteric/zwitterionic surfactants. Non-limiting examples of surfactants that may be used are provided below.

### Cationic Surfactants

The term "cationic surfactant" means a surfactant that is positively charged when it is contained in the composition according to the dislcosure. This surfactant may bear one or more positive permanent charges or may contain one or more functions that are cationizable in the composition according to the disclosure.

Non-limiting examples of cationic surfactants include behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quaternium-5), dodecyl dimethyl ethylbenzyl ammonium chloride(Quaternium-14), Quaternium-22, Quaternium-26, Quaternium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCI, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

The cationic surfactant(s) may be chosen from optionally polyoxyalkylenated, primary, secondary or tertiary fatty amines, or salts thereof, and quaternary ammonium salts, and mixtures thereof.

The fatty amines generally comprise at least one C₈-C₃₀ hydrocarbon-based chain.

Examples of quaternary ammonium salts that may especially be mentioned include: those corresponding to the general formula (III) below: 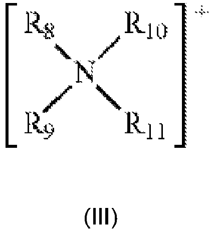 in which the groups R₈ to R₁₁, which may be identical or different, represent a linear or branched, saturated or unsaturated aliphatic group comprising from 1 to 30 carbon atoms, or an aromatic group such as aryl or alkylaryl, at least one of the groups R₈ to R₁₁ denoting a group comprising from 8 to 30 carbon atoms and preferably from 12 to 24 carbon atoms. The aliphatic groups may comprise heteroatoms especially such as oxygen, nitrogen, sulfur and halogens. The aliphatic groups are chosen, for example, from C₁-C₃₀ alkyl, C₂-C₃₀ alkenyl, C₁-C₃₀ alkoxy, polyoxy(C₂-C₆)alkylene, C₁-C₃₀ alkylamide, (C₁₂-C₂₂)alkylamido(C₂-C₆)alkyl, (C₁₂-C₂₂)alkyl acetate and C₁-C₃₀ hydroxyalkyl groups; X⁻ is an anion chosen from the group of halides, phosphates, acetates, lactates, (C₁-C₄)alkyl sulfates, and (C₁-C₄)alkyl- or (C₁-C₄)alkylarylsulfonates.

Among the quaternary ammonium salts of formula (III), those that are preferred are, on the one hand, tetraalkylammonium salts, for instance dialkyldimethylammonium or alkyltrimethylammonium salts in which the alkyl group contains approximately from 12 to 22 carbon atoms, in particular behenyltrimethylammonium, distearyldimethylammonium, cetyltrimethylammonium or benzyldimethylstearylammonium salts, or, on the other hand, oleocetyldimethylhydroxyethylammonium salts, palmitylamidopropyltrimethylammonium salts, stearamidopropyltrimethylammonium salts and stearamidopropyldimethylcetearylammonium salts.

In some cases it is useful to use salts such as the chloride salts of the following compounds:
A. a quaternary ammonium salt of imidazoline, such as, for example, those of formula (IV) below: 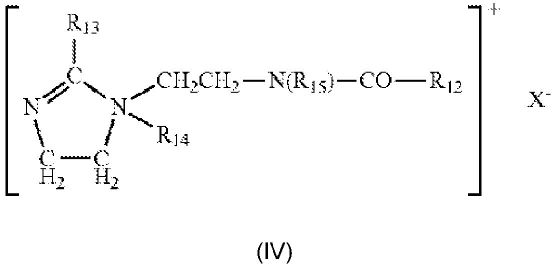 in which R₁₂ represents an alkenyl or alkyl group comprising from 8 to 30 carbon atoms, derived for example from tallow fatty acids, R₁₃ represents a hydrogen atom, a C₁-C₄ alkyl group or an alkyl or alkenyl group comprising from 8 to 30 carbon atoms, R₁₄ represents a C₁-C₄ alkyl group, R₁₅ represents a hydrogen atom or a C₁-C₄ alkyl group, X⁻ is an anion chosen from the group of halides, phosphates, acetates, lactates, alkyl sulfates, alkyl- or alkylaryl-sulfonates in which the alkyl and aryl groups preferably comprise, respectively, from 1 to 20 carbon atoms and from 6 to 30 carbon atoms. R₁₂ and R₁₃ preferably denote a mixture of alkenyl or alkyl groups containing from 12 to 21 carbon atoms, derived for example from tallow fatty acids, R₁₄ preferably denotes a methyl group, and R₁₅ preferably denotes a hydrogen atom. Such a product is sold, for example, under the name Rewoquat(R) W 75 by the company Rewo;
B. a quaternary diammonium or triammonium salt, in particular of formula (V): 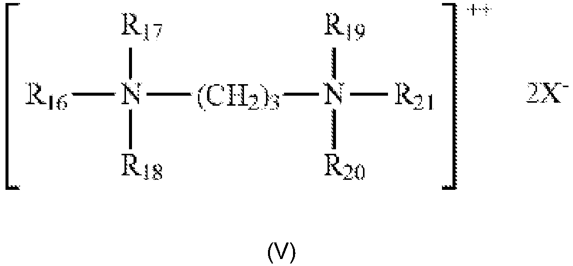 in which R₁₆ denotes an alkyl radical comprising approximately from 16 to 30 carbon atoms, which is optionally hydroxylated and/or interrupted with one or more oxygen atoms, R₁₇ is chosen from hydrogen or an alkyl radical comprising from 1 to 4 carbon atoms or a group (R₁₆ₐ)(R₁₇ₐ)(R₁₈ₐ)N-(CH₂)₃,
   R₁₆ₐ, R₁₇ₐ, R₁₈ₐ, R₁₈, R₁₉, R₂₀ and R₂₁, which may be identical or different, being chosen from hydrogen and an alkyl radical comprising from 1 to 4 carbon atoms, and X⁻ is an anion chosen from the group of halides, acetates, phosphates, nitrates and methyl sulfates. Such compounds are, for example, Finquat CT-P, sold by the company Finetex (Quaternium 89), and Finquat CT, sold by the company Finetex (Quaternium 75),
C. a quaternary ammonium salt containing at least one ester function, such as those of formula (VI) below: 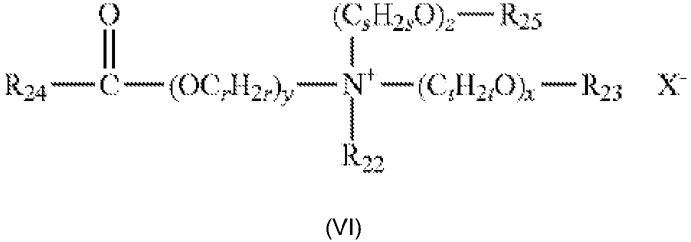 in which:
   R₂₂ is chosen from C₁-C₆ alkyl groups and C₁-C₆ hydroxyalkyl or dihydroxyalkyl groups;
   R₂₃ is chosen from: 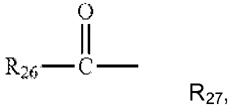 which is a linear or branched, saturated or unsaturated C₁-C₂₂ hydrocarbon-based group, and a hydrogen atom,
   R₂₅ is chosen from: 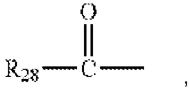
   R₂₉, which is a linear or branched, saturated or unsaturated C₁-C₆ hydrocarbon-based group, and a hydrogen atom,
   R₂₄, R₂₆ and R₂₈, which may be identical or different, are chosen from linear or branched, saturated or unsaturated C₇-C₂₁ hydrocarbon-based groups;
   r, s and t, which may be identical or different, are integers ranging from 2 to 6;
   y is an integer ranging from 1 to 10;
   x and z, which may be identical or different, are integers ranging from 0 to 10;
   X⁻ is a simple or complex, organic or mineral anion;
   with the proviso that the sum x+y+z is from 1 to 15, that when x is 0 then Rₙ denotes R₂₇, and that when z is 0 then R₂₅ denotes R₂₉.

The alkyl groups R₂₂ may be linear or branched, and more particularly linear. In some cases, R₂₂ denotes a methyl, ethyl, hydroxyethyl or dihydroxypropyl group, and more particularly a methyl or ethyl group. Advantageously, the sum x+y+z is from 1 to 10.

When R₂₃ is a hydrocarbon-based group R₂₇, it may be long and contain from 12 to 22 carbon atoms, or may be short and contain from 1 to 3 carbon atoms. When R₂₅ is an R₂₉ hydrocarbon-based group, it preferably contains 1 to 3 carbon atoms. Advantageously, R₂₄, R₂₅ and R₂₈, which may be identical or different, are chosen from linear or branched, saturated or unsaturated C₁₁-C₂₁ hydrocarbon-based groups, and more particularly from linear or branched, saturated or unsaturated C₁₁-C₂₁ alkyl and alkenyl groups.

In some cases, x and z, which may be identical or different, have values of 0 or 1. Likewise, in some cases y is equal to 1. In some cases, r, s and t, which may be identical or different, are equal to 2 or 3, and even more particularly are equal to 2.

The anion X⁻ is may be a halide (chloride, bromide or iodide) or an alkyl sulfate, more particularly methyl sulfate. However, use may be made of methanesulfonate, phosphate, nitrate, tosylate, an anion derived from an organic acid, such as acetate or lactate, or any other anion compatible with the ammonium containing an ester function.

The anion X⁻ is even more particularly chloride or methyl sulfate.

Use is made more particularly, in the composition according to the invention, of the ammonium salts of formula (VI) in which:
R₂₂ denotes a methyl or ethyl group,
x and y are equal to 1;
z is equal to 0 or 1;
r, s and t are equal to 2;
R₂₃ is chosen from: 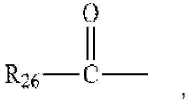 methyl, ethyl or C₁₄-C₂₂ hydrocarbon-based groups, and a hydrogen atom;
R₂₅ is chosen from: 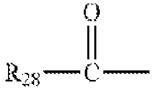 and a hydrogen atom;
R₂₄, R₂₆ and R₂₈, which may be identical or different, are chosen from linear or branched, saturated or unsaturated C₁₃-C₁₇ hydrocarbon-based groups, and preferably from linear or branched, saturated or unsaturated C₁₃-C₁₇ alkyl and alkenyl groups. The hydrocarbon-based groups are advantageously linear.

Mention may be made, for example, of the compounds of formula (VI) such as the diacyloxyethyldimethylammonium, diacylo xyethylhydroxyethylmethylammonium, monoacyloxyethyldihydroxyethylmethylammonium, triacyloxyethylmethylammonium and monoacyloxyethylhydroxyethyldimethylammonium salts (chloride or methyl sulfate in particular), and mixtures thereof. The acyl groups preferably contain 14 to 18 carbon atoms and are obtained more particularly from a plant oil, such as palm oil or sunflower oil. When the compound contains several acyl groups, these groups may be identical or different.

These products are obtained, for example, by direct esterification of triethanolamine, triisopropanolamine, an alkyldiethanolamine or an alkyldiisopropanolamine, which are optionally oxyalkylenated, with C₁₀-C₃₀ fatty acids or with mixtures of C₁₀-C₃₀ fatty acids of plant or animal origin, or by transesterification of the methyl esters thereof. This esterification is followed by quaternization using an alkylating agent such as an alkyl (preferably methyl or ethyl) halide, a dialkyl (preferably methyl or ethyl) sulfate, methyl methanesulfonate, methyl para-toluenesulfonate, glycol chlorohydrin or glycerol chlorohydrin. Such compounds are, for example, sold under the names Dehyquart(R) by the company Henkel, Stepanquat(R) by the company Stepan, Noxamium(R) by the company Ceca or Rewoquat(R) WE 18 by the company Rewo-Witco.

The composition according to the invention may contain, for example, a mixture of quaternary ammonium monoester, diester and triester salts with a weight majority of diester salts.

Use may be made of behenoylhydroxypropyltrimethylammonium chloride, provided by Kao under the name Quatarmin BTC 131.

### Nonionic Surfactants

Examples of nonionic surfactants that may be used are described, for example, in the Handbook of Surfactants by M. R. Porter, published by Blackie & Son (Glasgow and London), 1991, pp. 1 16. The nonionic surfactant may be alcohols, alpha-diols and (C₁-C₂₄)alkylphenols, these compounds being polyethoxylated, polypropoxylated and/or polyglycerolated, and containing at least one fatty chain comprising, for example, from 8 to 18 carbon atoms, it being possible for the number of ethylene oxide and/or propylene oxide groups to especially range from 2 to 50, and for the number of glycerol groups to especially range from 2 to 30.

Mention may also be made of copolymers of ethylene oxide and propylene oxide, optionally oxyethylenated sorbitan fatty acid esters, sucrose fatty acid esters, polyoxyalkylenated fatty acid esters, polyoxyalkylenated fatty amides, optionally oxyalkylenated alkyl(poly)glucosides, alkylglucoside esters, derivatives of N-alkylglucamine and of N-acylmethylglucamine, aldobionamides, amine oxides and (poly)oxyalkylenated silicones.

The nonionic surfactants are more particularly chosen from monooxyalkylenated or polyoxyalkylenated and monoglycerolated or polyglycerolated nonionic surfactants, and alkyl(poly)glucosides. The oxyalkylene units are more particularly oxyethylene or oxypropylene units, or a combination thereof, preferably oxyethylene units.

Useful nonionic surfactants may include : oxyalkylenated (C₈-C₂₄)alkylphenols; saturated or unsaturated, linear or branched, oxyalkylenated C₈-C₄₀ alcohols; saturated or unsaturated, linear or branched, oxyalkylenated C₈-C₃₀ amides; esters of saturated or unsaturated, linear or branched, C₈-C₃₀ acids and of polyethylene glycols; saturated or unsaturated, oxyethylenated plant oils; condensates of ethylene oxide and/or of propylene oxide, alone or as mixtures; oxyethylenated and/or oxypropylenated silicones; and alkyl(poly)glucosides.

As examples of monoglycerolated or polyglycerolated nonionic surfactants, monoglycerolated or polyglycerolated C₈-C₄₀ alcohols are useable. In particular, the monoglycerolated or polyglycerolated C C₈-C₄₀ alcohols correspond to formula (VIII) below:

R₂₉O-[CH₂-CH(CH₂OH)-O]*ₘ-*H (VIII)

in which formula (VIII):
R₂₉ represents a linear or branched C₈-C₄₀ and preferably C₈-C₃₀ alkyl or alkenyl radical; and
m represents a number ranging from 1 to 30, or from 1 to 10.

As examples of compounds of formula (VIII), mention may be made of lauryl alcohol containing 4 mol of glycerol (INCI name: Polyglyceryl-4 Lauryl Ether), lauryl alcohol containing 1.5 mol of glycerol, oleyl alcohol containing 4 mol of glycerol (INCI name: Polyglyceryl-4 Oleyl Ether), oleyl alcohol containing 2 mol of glycerol (INCI name: Polyglyceryl-2 Oleyl Ether), cetearyl alcohol containing 2 mol of glycerol, cetearyl alcohol containing 6 mol of glycerol, oleocetyl alcohol containing 6 mol of glycerol, and octadecanol containing 6 mol of glycerol.

The alcohol of formula (VIII) may represent a mixture of alcohols in the same way that the value of m represents a statistical value, which means that, in a commercial product, several species of polyglycerolated fatty alcohols may coexist in the form of a mixture.

The alkyl(poly)glycoside nonionic surfactant(s) may be represented by formula (IX) below:

R₃₀O-(R₃₁O)*ₜ*(G)*ᵥ* (IX)

in which:
R₃₀ represents a saturated or unsaturated, linear or branched alkyl group comprising from about 8 to 24 carbon atoms, or an alkylphenyl group in which the linear or branched alkyl group comprises from 8 to 24 carbon atoms;
R₃₁ represents an alkylene group containing from about 2 to 4 carbon atoms,
G represents a saccharide unit comprising from 5 to 6 carbon atoms,
t denotes a value ranging from 0 to 10, or from 0 to 4, and
v denotes a value ranging from 1 to 15.

In some cases, the alkyl(poly)glycoside nonionic surfactant(s) correspond to formula (IX) in which:
R₃₀ denotes a linear or branched, saturated or unsaturated alkyl group containing from 8 to 18 carbon atoms,
G denotes glucose, fructose or galactose, preferably glucose,
t denotes a value ranging from 0 to 3, and is preferably equal to 0, and
R₃₁ and v are as defined previously.

The degree of polymerization of the alkyl(poly)glucoside nonionic surfactant(s), as represented, for example, by the index v in formula (IX), ranges on average from 1 to 15, or from 1 to 4. This degree of polymerization more particularly ranges from 1 to 2 and better still from 1.1 to 1.5, on average.

The glycoside bonds between the saccharide units are of 1.6 or 1.4 type and preferably of 1.4 type.

Examples of compounds of formula (IX) that may especially be mentioned are the products sold by the company Cognis under the names Plantaren® (600 CS/U, 1200 and 2000) or Plantacare ® (818, 1200 and 2000). Use may also be made of the products sold by the company SEPPIC under the names Triton CG 110 (or Oramix CG 110) and Triton CG 312 (or Oramix® NS 10), the products sold by the company BASF under the name Lutensol GD 70 or the products sold by the company Chem Y under the name AG10 LK. Use may also be made, for example, of the 1,4-(C₈-C₁₆) alkylpolyglucoside as an aqueous solution at 53% by weight relative to the total weight of the solution, sold by Cognis under the reference Plantacare 818 UP.

### Amphoteric or Zwitterionic Surfactants

The amphoteric or zwitterionic surfactant that may be used in compositions according to the disclosure may be derivatives of aliphatic secondary or tertiary amines, optionally quaternized, in which derivatives the aliphatic group is a linear or branched chain comprising from 8 to 22 carbon atoms, the amine derivatives containing at least one anionic group, such as a carboxylate, sulfonate, sulfate, phosphate or phosphonate group. Mention may be made in particular of (C₈-C₂₀)alkylbetaines such as cocoylbetaine, sulfobetaines, (C₈-C₂₀)alkylamido(C₂-C₈)alkylbetaines such as cocoylamidopropylbetaine or (C₈-C₂₀)alkylamido(C₆-C₈)-alkylsulfobetaines, and mixtures thereof.

Among the derivatives of aliphatic secondary or tertiary amines, optionally quaternized, that may be used, as defined above, mention may also be made of the compounds of respective structures (I), (II) and (IIa) below:

Ra-C(O)-NHCH₂CH₂-N⁺(Rb)(Rc)-CH₂COO⁻,M⁺, X⁻ (I)

in which formula (I):
Ra represents a C₁₀-C₃₀ alkyl or alkenyl group derived from an acid Ra-COOH preferably present in hydrolysed coconut oil, or a heptyl, nonyl or undecyl group; Rb represents a beta-hydroxyethyl group; and
Rc represents a carboxymethyl group;
M⁺ represents a cationic counterion derived from an alkali metal or alkaline-earth metal, such as sodium, an ammonium ion or an ion derived from an organic amine; and
X⁻ represents an organic or mineral anionic counterion, such as that chosen from halides, acetates, phosphates, nitrates, (C₁-C₄)alkyl sulfates, (C₁-C₄)alkyl- or (C₁-C₄)alkylarylsulfonates, in particular methyl sulfate and ethyl sulfate; or alternatively M⁺ and X⁻ are absent;

   Ra'-C(O)-NHCH₂CH₂-N(B)(B') (II)

   in which formula (II):
   B represents the group -CH₂-CH₂-O-X';
   B' represents the group -(CH²)_{z}Y', with z=1 or 2;
   X' represents the group -CH₂-COOH, CH₂-COOZ', -CH₂CH₂-COOH or-CH₂CH₂-COOZ', or a hydrogen atom;
   Y' represents the group -COOH, -COOZ', CH₂CH(OH)SO₃H or the group - CH₂CH(OH)SO₃Z';
   Z' represents a cationic counterion derived from an alkali metal or alkaline-earth metal, such as sodium, an ammonium ion or an ion derived from an organic amine;
   Ra' represents a C₁₀-C₃₀ alkyl or alkenyl group of an acid Ra'-COOH, which may be coconut oil or in hydrolysed linseed oil, or an alkyl group, especially a C₁₇ group and its iso form, or an unsaturated C₁₇ group.

The compounds of this type are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid and cocoamphodipropionic acid.

By way of example, mention may be made of the cocoamphodiacetate sold by the company Rhodia under the trade name Miranol C2M Concentrate and the cocoamphodipropionate sold by the company Evonik Goldschmidt under the trade name Rewoteric AM KSF 40.

Ra"-NH-CH(Y")-(CH₂)*ₙ-*C(O)-NH-(CH₂)*_{n'}-* N(Rd)(Re) (IIa)

in which formula (IIa):
Y" represents the group -COOH, -COOZ", -CH₂CH(OH)SO₃H or the group - CH₂CH(OH)SO₃Z";
Rd and Re, independently of each other, represent a C₁-C₄ alkyl or hydroxyalkyl radical;
Z" represents a cationic counterion derived from an alkali metal or alkaline-earth metal, such as sodium, an ammonium ion or an ion derived from an organic amine;
Ra" represents a C₁₀-C₃₀ alkyl or alkenyl group of an acid Ra"-COOH;
n and n' denote, independently of each other, an integer ranging from 1 to 3; and mixtures of these compounds.

Among the compounds of formula (IIa), mention may be made of the compound classified in the CTFA dictionary under the name sodium diethylaminopropyl cocoaspartamide and sold by the company Chimex under the name Chimexane HB. In some instances, the amphoteric or zwitterionic surfactant(s) are chosen from cocoylbetaine, cocoylamidopropylbetaine and sodium cocoylamidoethyl-N-hydro xyethylaminopropionate.

The term "anionic surfactant" means a surfactant comprising, as ionic or ionizable groups, only anionic groups. These anionic groups are chosen preferably from the groups CO₂H, CO₂⁻, SO₃H, SO₃⁻, OSO₃H, OSO₃⁻ O₂PO₂H, O₂PO₂H and O₂PO₂²⁻.

The anionic surfactant(s) that may be used may be alkyl sulfates, alkyl ether sulfates, alkylamido ether sulfates, alkylaryl polyether sulfates, monoglyceride sulfates, alkylsulfonates, alkylamide sulfonates, alkylarylsulfonates, alpha-olefin sulfonates, paraffin sulfonates, alkylsulfosuccinates, alkyl ether sulfosuccinates, alkylamide sulfosuccinates, alkyl sulfoacetates, acylsarcosinates, acylglutamates, alkylsulfosuccinamates, acylisethionates and N-acyltaurates, salts of alkyl monoesters and polyglycoside-polycarboxylic acids, acyllactylates, salts of D-galactoside uronic acids, salts of alkyl ether carboxylic acids, salts of alkyl aryl ether carboxylic acids, and salts of alkylamido ether carboxylic acids; or the non-salified forms of all of these compounds, the alkyl and acyl groups of all of these compounds containing from 6 to 24 carbon atoms and the aryl group denoting a phenyl group. Some of these compounds may be oxyethylenated and then preferably comprise from 1 to 50 ethylene oxide units.

The salts of C₆-C₂₄ alkyl monoesters of polyglycoside-polycarboxylic acids may be chosen from C₆-C₂₄ alkyl polyglycoside-citrates, C₆-C₂₄ alkyl polyglycoside-tartrates and C₆-C₂₄ alkyl polyglycoside-sulfo succinates.

When the anionic surfactant(s) are in salt form, they may be chosen especially from alkali metal salts such as the sodium or potassium salt and preferably the sodium salt, ammonium salts, amine salts and in particular amino alcohol salts, or alkaline-earth metal salts such as the magnesium salt.

Examples of amino alcohol salts that may especially be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.Alkali metal or alkaline-earth metal salts and in particular the sodium or magnesium salts may be used.

Use is also made of (C₆-C₂₄)alkyl sulfates, (C₆-C₂₄)alkyl ether sulfates, which are optionally ethoxylated, comprising from 2 to 50 ethylene oxide units, and mixtures thereof, in particular in the form of alkali metal salts or alkaline-earth metal salts, ammonium salts or amino alcohol salts. More preferentially, the anionic surfactant(s) are chosen from (C₁₀-C₂₀)alkyl ether sulfates, and in particular sodium lauryl ether sulfate containing 2.2 mol of ethylene oxide.

Implementation of the present disclosure is provided by way of the following examples.

### Example 1

### (Formulations)

Composition (B) according to the instant disclosure is presented in the table below. Compositions A and C fall outside the scope of the invention as claimed and are reference examples.

| | | **A** | **B** | **C** |
|---|---|---|---|---|
| COMPONENT | US INCI NAME | AMOUNT | AMOUNT | AMOUNT |
| ACTIVE | SULFUR | 18 | 10 | 3 |
| | NIACINAMIDE | 3 | 4 | 15 |
| | PHENYLETHYL RESORCINOL | 0.5 | 0.5 | 3 |
| | GLYCOLIC ACID | 3.9 | 3.7 | 0.5 |
| | ALLANTOIN | 1.0 | 0.1 | 0.1 |
| FATTY COMPOUNDS | HYDROGENATED LECITHIN + STEARYL ALCOHOL + OCTYLDODECYL MYRISTATE | 5.3 | 4.4 | 4.0 |
| VITAMIN | VITAMIN E | 0.5 | 0.5 | 0.5 |
| THICKENERS | | 6.5 | 6.55 | 7.5 |
| PRESERVATIVES | | 1.2 | 1.2 | 1.0 |
| NONIONIC SURFACTANTS | GLYCERYL STEARATE + GLYCERYL STEARATE (and) PEG-100 STEARATE + CETEARYL ALCOHOL (and) CETEARETH-20 | 5.0 | 4.3 | 6.2 |
| VEGETABLE EXTRACTS | | 0.5 | 0.2 | 0.1 |
| pH ADJUSTER | SODIUM HYDROXIDE | 1.3 | 1.3 | 1.3 |
| SOLVENT | GLYCERIN | 5.0 | 2.0 | 3.0 |
| | WATER | q.s | q.s. | q.s. |

### Example 2

### (Evaluation)

Fifty-four women ages 18-45 years with normal-oily, oily, or acne-prone skin, with at least one post-acne mark completed a four week safety and non-comedogeneicity study under the supervision of a dermatologist. A composition from Example 1 was applied one to three times daily to the affected area on the face, avoiding contact with eyes or areas with broken skin. A dermatologist evaluation was conducted at baseline and after four weeks of use to determine lesion counts and tolerance. Consumer evaluations were also included in the study. The results of the consumer evaluation are presented in the table below.

| **Consumer Evaluation** | **Week 2** | **Week 4** |
|---|---|---|
| Visible pores appear reduced/minimized | 67.5% | 77.8% |
| Dark marks from acne appear to be reduced | 50.0% | 75.9% |

At week 2, 67.5 % of consumers reported that the appearance of visible pores was reduced; and at four weeks this number jumped to 77.8 % of consumers. With respect to the reduction of dark marks from acne, 50 % of consumers reported reduction at two weeks; and at four weeks this number jumped to 75.9%.

### Example 3

### (Clinical Testing)

Fifty subjects (male and female) ages 18-50 with mild to moderate acne (score 2-3 on Global Assesment Scale) or acne prone skin with at least one inflammatory lesion to track completed a seven day study. A composition of Example 1 was applied to the subject's skin up to three times per day. The skin was recruited based on the Global Assessment Scale, shown below.

| | | |
|---|---|---|
| 0 | Clear | Residual hyperpigmentation and erythema may be present |
| 1 | Almost Clear | A few scattered comedones and a few (less than five) small papules |
| 2 | Mild | Easily recognizable; less than half the face is involved. Many comedones and many papules and pustules. |
| 3 | Moderate | More than half of the face is involved. Numerous comedones, papules, and pustules. |
| 4 | Severe | Entire face is involved. Covered with comedones, numerous papules and pustules and few nodules and cysts. |
| 5 | Very Severe | Highly inflammatory acne covering the face; with nodules and cysts present. |

The results are presented in the following table.

| **Assessment** | **Time Point** | **n** | **Mean** | **Mean Change from Baseline** | **Mean % Change from Baseline** | **p-value** |
|---|---|---|---|---|---|---|
| Diameter (mm) | Baseline | 51 | 3.43 | | | |
| | Day 1 | 51 | 3.07 | -0.37 | -10.66% | <0.001 |
| | Day 2 | 51 | 2.56 | -0.87 | -25.44% | <0.001 |
| | Day 3 | 51 | 2.19 | -1.25 | -36.33% | <0.001 |
| | Day 7 | 51 | 1.38 | -2.05 | -59.67% | <0.001 |
| Height (mm) | Baseline | 51 | 1.34 | | | |
| | Day 1 | | 1.10 | -0.24 | -17.96% | <0.001 |
| | Day 2 | 51 | 0.77 | -0.57 | -42.74% | <0.001 |
| | Day 3 | 51 | 0.58 | -0.75 | -56.36% | <0.001 |
| | Day 7 | 51 | 0.21 | -1.13 | -84.58% | <0.001 |
| Redness | Baseline | 51 | 5.19 | | | |
| | Day 1 | 51 | 5.11 | -0.08 | -1.54% | 0.290 |
| | Day 2 | 51 | 4.78 | -0.40 | -7.90% | 0.005 |
| | Day 3 | 51 | 4.46 | -0.73 | -14.07% | <0.001 |
| | Day 7 | 51 | 3.29 | -1.89 | -36.61 % | <0.001 |

The data show a significant decrease (improvement) in diameter and height for all time asses compared to baseline. Redness showed statistical and clinically significant improvement on day 2, 3, and 7 compared to baseline.

Consumer evaluations were also included in the study for which the results are provided below.

| | **Day 1** | **Day 2** | **Day 3** | **Day 7** |
|---|---|---|---|---|
| Skin does NOT appear dry/flaky. | 82.4 | 80.4 | 82.4 | 82.4 |
| Existing blemishes appear less visible. | 60.8 | 76.5 | 80.4 | 92.2 |
| Existing blemishes appear less red. | 66.7 | 82.4 | 80.4 | 90.2 |
| Skin appears more clear. | 56.9 | 74.5 | 80.4 | 88.2 |
| Size of existing blemishes appear reduced. | 58.8 | 82.4 | 86.3 | 96.1 |
| New breakouts do not appear to be occurring where test product has been applied. | 80.4 | 84.3 | 90.2 | 94.1 |
| Overall skin quality appears improved. | 76.5 | 82.4 | 92.2 | 92.2 |
| Overall, skin feels improved. | 70.6 | 80.4 | 92.2 | 90.2 |

As used herein, the terms "comprising," "having," and "including" are used in their open, non-limiting sense.

The terms "a," "an," and "the" are understood to encompass the plural as well as the singular.

The expression "at least one" means one or more and thus includes individual components as well as mixtures/combinations.

All ranges and values disclosed herein are inclusive and combinable. For examples, any value or point described herein that falls within a range described herein can serve as a minimum or maximum value to derive a sub-range, etc.

## Claims

1. A cosmetic composition comprising:
(a) a cosmetically acceptable carrier comprising water and one or more cosmetically acceptable organic solvents; and
(b) a combination of skin-active ingredients comprising:
i. 5 wt.% to 15 wt.% of sulfur;
ii. 1 wt.% to 10 wt.% of niacinamide;
iii. 0.1 wt.% to 2 wt.% of phenylethyl resorcinol;
iv. glycolic acid;
v. allantoin; and
(c) one or more nonionic surfactants, wherein the total amount of the one or more nonionic surfactants of (c) is from 0.1 wt.% to 15 wt.%, based on the total weight of the cosmetic composition.

2. The cosmetic composition of claim 1, wherein the one or more nonionic surfactants are selected from the group consisting of a polyol ester, a glycerol ether, an oxyethylenated ether, an oxypropylenated ether, and an ethylene glycol polymer.

3. The cosmetic composition of claim 2, wherein the one or more nonionic surfactants of (c) comprise a combination of a polyol ester and an ethylene glycol polymer,

4. The cosmetic composition of claim 2, wherein the one or more nonionic surfactants of (c) comprise a combination of glyceryl stearate, PEG-100 stearate, and ceteareth-20.

5. The cosmetic composition of claim 2, wherein the one or more nonionic surfactants of (c) comprise one or more fatty alcohols, the one or more fatty alcohols are selected from the group consisting of caproic alcohol, caprylic alcohol, 2-ethylhexyl alcohol, capric alcohol, lauryl alcohol, isotridecyl alcohol, myristyl alcohol, cetyl alcohol, hexyl decanol, palmitoleyl alcohol, stearyl alcohol, cetearyl alcohol, isostearyl alcohol, oleyl alcohol, elaidyl alcohol, petroselinyl alcohol, linolyl alcohol, linolenyl alcohol, elaeostearyl alcohol, arachyl alcohol, gadoleyl alcohol, behenyl alcohol, octyl dodecanol, erucyl alcohol brassidyl alcohol, coconut oil, and behenyl alcohol.

6. The cosmetic composition of any one of the above claims, further comprising:
(d) one or more vitamins, the one or more vitamins being preferably selected from the group consisting of ascorbic acid, vitamin A, vitamin E, and vitamin B₅.

7. The cosmetic composition of claim 6, wherein the total amount of the one or more vitamins is from 0.01 wt.% to 5 wt.%.

8. The cosmetic composition of any one of the above claims, wherein the one or more cosmetically acceptable organic solvents are selected from the group consisting of ethylene glycol, propylene glycol, butylene glycol, hexylene glycol, propane diol, and glycerin.

9. A composition according to any one of the above claims for use in a method for providing anti-inflammatory and/or antibacterial benefits to the skin.

10. A composition according to any one of the above claims for use in a method for treating acne.

11. A non-therapeutic cosmetic method for improving the appearance of the skin comprising applying a composition of any one of the above claims to the skin.

## Patentansprüche

1. Kosmetische Zusammensetzung, umfassend:
(a) einen kosmetisch annehmbaren Träger, umfassend Wasser und ein oder mehrere kosmetisch annehmbare organische Lösungsmittel; und
(b) eine Kombination von hautaktiven Bestandteilen, umfassend:
i. 5 Gew.-% bis 15 Gew.-% Schwefel;
ii. 1 Gew.-% bis 10 Gew.-% Niacinamid;
iii. 0,1 Gew.-% bis 2 Gew.-% Phenylethylresorcin;
iv. iv. Glykolsäure;
v. Allantoin; und
(c) ein oder mehrere nichtionische Tenside, wobei die Gesamtmenge des einen oder der mehreren nichtionischen Tenside von (c) von 0,1 Gew.-% bis 15 Gew.-%, bezogen auf das Gesamtgewicht der kosmetischen Zusammensetzung, ist.

2. Kosmetische Zusammensetzung nach Anspruch 1, wobei das eine oder die mehreren nichtionischen Tenside ausgewählt sind aus der Gruppe bestehend aus einem Polyolester, einem Glycerinether, einem oxyethylenierten Ether, einem oxypropylenierten Ether und einem Ethylenglycolpolymer.

3. Kosmetische Zusammensetzung nach Anspruch 2, wobei das eine oder die mehreren nichtionischen Tenside von (c) eine Kombination aus einem Polyolester und einem Ethylenglykolpolymer umfassen,

4. Kosmetische Zusammensetzung nach Anspruch 2, wobei das eine oder die mehreren nichtionischen Tenside von (c) eine Kombination von Glycerylstearat, PEG-100-Stearat und Ceteareth-20 umfassen.

5. Kosmetische Zusammensetzung nach Anspruch 2, wobei das eine oder die mehreren nichtionischen Tenside von (c) einen oder mehrere Fettalkohole umfassen, wobei der eine oder die mehreren Fettalkohole ausgewählt sind aus der Gruppe bestehend aus Capronsäurealkohol, Caprylalkohol, 2-Ethylhexylalkohol, Caprinsäurealkohol, Laurylalkohol, Isotridecylalkohol, Myristylalkohol, Cetylalkohol, Hexyldekanol, Palmitoleylalkohol, Stearylalkohol, Cetearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Octyldodekanol, Erucylalkohol, Brassidylalkohol, Kokosnussöl und Behenylalkohol.

6. Kosmetische Zusammensetzung nach einem der obigen Ansprüche, ferner umfassend:
(d) ein oder mehrere Vitamine, wobei das eine oder die mehreren Vitamine vorzugsweise ausgewählt sind aus der Gruppe bestehend aus Ascorbinsäure, Vitamin A, Vitamin E und Vitamin B₅.

7. Kosmetische Zusammensetzung nach Anspruch 6, wobei die Gesamtmenge des einen oder der mehreren Vitamine von 0,01 Gew.-% bis 5 Gew.-% ist.

8. Kosmetische Zusammensetzung nach einem der obigen Ansprüche, wobei das eine oder die mehreren kosmetisch annehmbaren organischen Lösungsmittel ausgewählt sind aus der Gruppe bestehend aus Ethylenglykol, Propylenglykol, Butylenglykol, Hexylenglykol, Propandial und Glycerin.

9. Zusammensetzung nach einem der obigen Ansprüche zur Verwendung in einem Verfahren zum Bereitstellen von entzündungshemmendem und/oder antibakteriellem Nutzen für die Haut.

10. Zusammensetzung nach einem der obigen Ansprüche zur Verwendung in einem Verfahren zum Behandeln von Akne.

11. Nicht-therapeutisches kosmetisches Verfahren zum Verbessern des Aussehens der Haut, umfassend ein Auftragen einer Zusammensetzung nach einem der obigen Ansprüche auf die Haut.

## Revendications

1. Composition cosmétique comprenant :
(a) un support cosmétiquement acceptable comprenant de l'eau et un ou plusieurs solvants organiques cosmétiquement acceptables ; et
(b) une association de substances actives sur la peau comprenant :
i. de 5 % en poids à 15 % en poids de soufre ;
ii. de 1 % en poids à 10 % en poids de niacinamide ;
iii. de 0,1 % en poids à 2 % en poids de phényléthyl-résorcinol ;
iv. de l'acide glycolique ;
v. de l'allantoïne ; et
(c) un ou plusieurs tensioactifs non-ioniques, dans laquelle la quantité totale du ou des tensioactifs non-ioniques de (c) est comprise entre 0,1 % en poids et 15 % en poids, sur la base du poids total de la composition cosmétique.

2. Composition cosmétique selon la revendication 1, dans laquelle le ou les tensioactifs non-ioniques sont choisis dans le groupe constitué d'un ester de polyol, d'un éther de glycérol, d'un éther oxyéthyléné, d'un éther oxypropyléné et d'un polymère d'éthylène glycol.

3. Composition cosmétique selon la revendication 2, dans laquelle le ou les tensioactifs non-ioniques de (c) comprennent une association d'un ester de polyol et d'un polymère d'éthylène glycol.

4. Composition cosmétique selon la revendication 2, dans laquelle le ou les tensioactifs non-ioniques de (c) comprennent une association de stéarate de glycéryle, de stéarate de PEG-100 et de cétéareth-20.

5. Composition cosmétique selon la revendication 2, dans laquelle le ou les tensioactifs non-ioniques de (c) comprennent un ou plusieurs alcools gras, le ou les alcools gras sont choisis dans le groupe constitué d'alcool caproïque, d'alcool caprylique, d'alcool 2-éthylhexylique, d'alcool caprique, d'alcool laurylique, d'alcool isotridécylique, d'alcool myristylique, d'alcool cétylique, d'hexyldécanol, d'alcool palmitoléylique, d'alcool stéarylique, d'alcool cétéarylique, d'alcool isostéarylique, d'alcool oléylique, d'alcool élaidylique, d'alcool pétrosélinylique, d'alcool linolylique, d'alcool linolénylique, d'alcool élaéostéarylique, d'alcool arachidylique, d'alcool gadoléylique, d'alcool béhénylique, d'octyl dodécanol, d'alcool érucylique, d'alcool brassidylique, d'huile de noix de coco et d'alcool béhénylique.

6. Composition cosmétique selon l'une quelconque des revendications précédentes, comprenant en outre :
(d) une ou plusieurs vitamines, la ou les vitamines étant de préférence choisies dans le groupe constitué d'acide ascorbique, de vitamine A, de vitamine E et de vitamine B₅.

7. Composition cosmétique selon la revendication 6, dans laquelle la quantité totale de la ou des vitamines est comprise entre 0,01 % en poids et 5 % en poids.

8. Composition cosmétique selon l'une quelconque des revendications précédentes, dans laquelle le ou les solvants organiques cosmétiquement acceptables sont choisis dans le groupe constitué d'éthylène glycol, de propylène glycol, de butylène glycol, d'hexylène glycol, de propane diol et de glycérine.

9. Composition selon l'une quelconque des revendications précédentes destinée à être utilisée dans un procédé procurant des bienfaits anti-inflammatoires et/ou anti-bactériens à la peau.

10. Composition selon l'une quelconque des revendications précédentes destinée à être utilisée dans un procédé de traitement de l'acné.

11. Procédé cosmétique non-thérapeutique permettant d'améliorer l'apparence de la peau comprenant l'application sur la peau d'une composition selon l'une quelconque des revendications précédentes.
